# EUROPÄISCHE PATENTSCHRIFT

(11) **EP 2 162 456 B1**
(45) Veröffentlichungstag und Bekanntmachung des Hinweises auf die Patenterteilung: **06.07.2011**
(21) Anmeldenummer: 08748372.3
(22) Anmeldetag: 29.05.2008
(51) Int. Cl.: C07D 475/04

(54) **Stabile kristalline (6S) -N (5) -Methyl-5,6,7,8-tetrahydrofolsäure, deren Herstellung und Verwendung**
Stable crystalline (6S)-N(5)-methyl-5,6,7,8-tetrahydrofolic acid, process for its preparation and its use
Acide (6S)-N-(5)-méthyl-5,6,7,8-tétrahydrofolique stable et cristallin, procédé pour sa préparation et son utilisation

(30) Priorität: 30.05.2007 CH 856072007
(43) Veröffentlichungstag der Anmeldung: 17.03.2010
(60) Teilanmeldung: 11003589.6
(73) Patentinhaber: Cerbios-Pharma S.A., CH-6917 Barbengo (CH)
(72) Erfinder: MANZOTTI, Raffella, I-22020 Pellio Intelvi (Como) (IT); MOROSOLI, Moreno, CH-6950 Tesserete (CH)
(74) Vertreter: Zink-Wild, Markus Peter
(86) Internationale Anmeldenummer: PCT/CH2008/000242
(87) Internationale Veröffentlichungsnummer: WO 2008/144953

(56) Entgegenhaltungen:
- EP-A- 0 455 013
- EP-A- 1 044 975

## Beschreibung

Die vorliegende Erfindung betrifft stabile, kristalline (6S)-N(5)-Methyl-5,6,7,8-tetrahydrofolsäure der Formel IV und ein Verfahren zu deren Herstellung.

In der vorliegenden Erfindung wird für 5,6,7,8-Tetrahydrofolsäure manchmal die Abkürzung THF verwendet.

Eine kurze Zusammenfassung über die pharmakologische Bedeutung von N(5)-Methyl-5,6,7,8-tetrahydrofolsäure - hierin teilweise mit N(5)-Methyl-THF abgekürzt - und Derivaten davon ist in der Beschreibungseinleitung von EP 0 455 013 A1 gegeben. Im gleichen Dokument wird auch auf die Bedeutung der individuellen (6S)-und (6R)-Diastereoisomeren der N(5)-Methyl-THF hingewiesen. Ebenso wird hierin der Stand der Technik betreffend die Herstellung der reinen (6S)- und (6R)-Diastereoisomeren der N(5)-Methyl-THF beschrieben.

In EP 1 044 975 A1 werden stabile kristalline Salze von N(5)-Methyl-THF beschrieben. Ebenso werden hierin auch kristalline Calciumsalze von (6S)-N(5)-Methyl-THF beschrieben, wobei diese Salze im entsprechenden Pulverröntgendiagramm klar definierte 2-Theta-Werte haben.

Beim Herstellungsverfahren dieser Salze werden als Ausgangsmaterialien entweder das (6RS)-Diastereoisomerengemisch oder die bereits aufgetrennten (6S)-oder (6R)-Diastereoisomeren verwendet. Dieses Verfahren beinhaltet eine Temperaturbehandlung von über 60°C, vorzugsweise von über 85°C, wobei in den Ausführungsbeispielen Temperaturen von 90°C bis 100°C erwähnt sind.

Eine solche Temperaturbehandlung ist selbstverständlich für eine industrielle Herstellung dieser Salze wenig geeignet.

In EP 1 044 975 A1 ist nicht angegeben, wie die einzelnen (6S)- oder (6R)-Diastereoisomeren von N(5)-Methyl-THF erhalten worden sind.

Es ist ein Ziel der vorliegenden Erfindung, stabile, kristalline (6S)-N(5)-Methyl-5,6,7,8-tetrahydrofolsäure der Formel IV und ein Verfahren zur Herstellung dieser Verbindung zur Verfügung zu stellen.

Mit der vorliegenden Erfindung wird dieses Ziel erreicht.

Das erfindungsgemässe Verfahren zur Herstellung von stabiler, kristalliner (6S)-N(5)-Methyl-5,6,7,8-tetrahydrofolsäure der Formel IV
ist dadurch gekennzeichnet, dass man
- zu einer wässrigen Lösung des Calciumsalzes von (6S)-N(5)-Methyl-5,6,7,8-tetrahydrofolsäure der Formel III mit einem Anteil des entsprechenden (6R)-Diastereoisomers von ≤ 2 Gew.-% entweder Essigsäure oder eine Sulfonsäure in einer solchen Menge hinzugibt, bis ein pH-Wert von 5,5 erhalten wird,
- die so erhaltene Lösung auf eine Temperatur im Bereich von 44°C bis 46°C erwärmt,
- zu dieser erwärmten Lösung entweder Essigsäure oder eine Sulfonsäure in einer solchen Menge hinzugibt, bis ein pH-Wert im Bereich von 4,3 bis 4,4 erhalten wird, wobei die Kristallisation von (6S)-N(5)-Methyl-5,6,7,8-tetrahydrofolsäure der Formel IV beginnt, wobei während dieser Kristallisation der pH-Wert im Bereich von.4,3 bis 4,4 durch kontinuierliche Hinzugabe von entweder Essigsäure oder einer Sulfonsäure aufrecht erhalten wird, und
- den so erhaltenen kristallinen Festkörper bei einer Temperatur im Bereich von 44°C bis 46°C abfiltriert und isoliert.

Bevorzugte Ausführungsformen der vorliegenden Erfindung sind in den abhängigen Ansprüchen definiert.

Im folgenden Teil werden mögliche Ausführungsformen der vorliegenden Erfindung beschrieben.

Dabei wird auch Bezug auf die Figuren genommen.

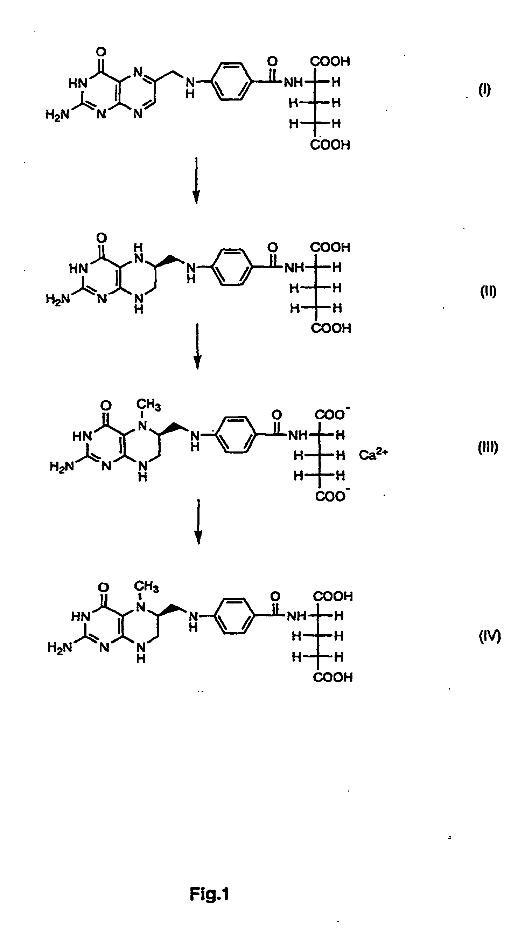

Figur 1 zeigt ein Reaktionsschema, beginnend mit der Verbindung der Formel I und endend mit der Verbindung der Formel IV.

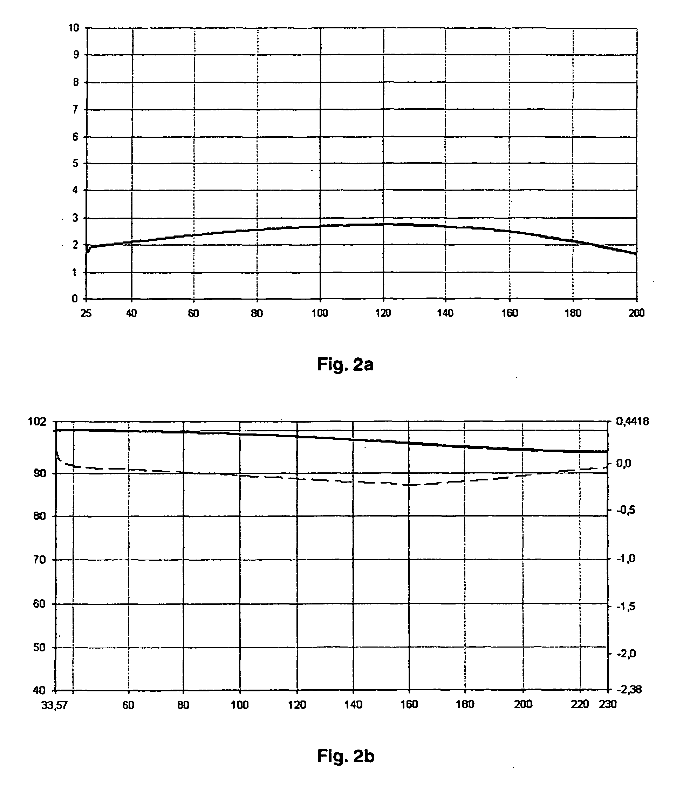

Figur 2a zeigt das "Differential Scanning Calorimetric" Profil, DSC, der Verbindung der Formel IV. Auf der Abszisse ist die Temperatur in °C aufgetragen, und auf der Ordinate ist der endotherme Wärmefluss in mW aufgetragen.
Figur 2b zeigt das thermogravimetrische Profil, TGA, der Verbindung der Formel IV. Auf der Abszisse ist die Temperatur in °C aufgetragen. Auf der linken Ordinate sind Gewichtsprozente und auf der rechten Ordinate ist die Ableitung dieser Gewichtsprozente gegenüber der Zeit t (Minuten) aufgetragen. Die ausgezogene Linie bezieht sich auf die linke Ordinate, und die gestrichelte Linie bezieht sich auf die rechte Ordinate.

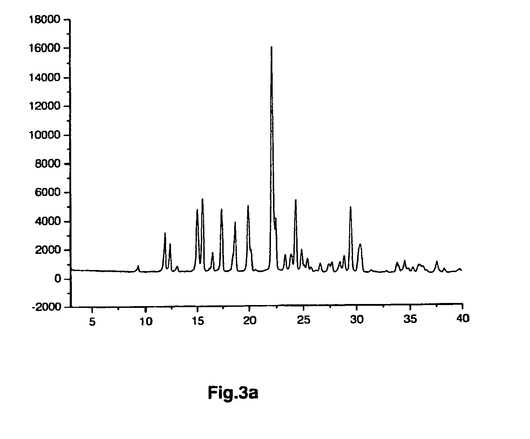

Figur 3a zeigt das Pulverröntgendiagramm der Verbindung der Formel IV. Auf der Abszisse sind die 2 Theta-Werte aufgetragen, und auf der Ordinate ist die Intensität (counts) aufgetragen.

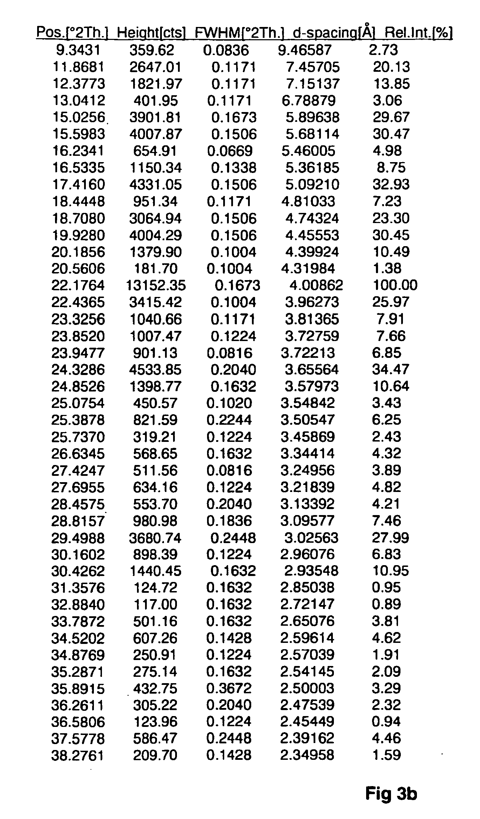

Figur 3b zeigt die 2 Theta-Werte mit einem Fehler von etwa ± 0,2 Grad des in Figur 3a gezeigten Pulverröntgendiagramms.

(6S)-5,6,7,8-Tetrahydrofolsäure der Formel II mit einem Anteil des entsprechenden (6R)-Diastereoisomers im Bereich von 4 Gew.-% bis 8 Gew.-% wurde gemäss EP 0 600 460 hergestellt.

Es war ganz überraschend, dass man eine wässrigen Lösung des Calciumsalzes von (6S)-N(5)-Methyl-5,6,7,8-tetrahydrofolsäure der Formel III mit einem erhöhten Gehalt des (6S)-Isomers erhalten konnte, und zwar durch selektives Auskristallisieren des 1:1 Gemisches der diastereoisomeren Salze aus der Lösung.

Routinemässig konnte man eine Lösung isolieren, welche einen Gehalt des (6S)-Isomers von wenigstens 98% hatte.

Es war auch ganz überraschend, die entsprechende stabile, kristalline freie Säure der Formel IV aus der oben beschriebenen Lösung zu erhalten: zudem wurde während dieses Schrittes eine weitere Erhöhung der diastereoisomeren Reinheit von wenigstens 99% erreicht.

Die stabile, kristalline (6S)-N(5)-Methyl-5,6,7,8-tetrahydrofolsäure der Formel IV kann in einer solchen diastereoisomeren Reinheit isoliert werden, dass diese Säure als wenigstens ein aktiver Bestandteil in einem Nährungsergänzungsmittel oder in einem Medikament verwendet werden kann.

Die vorliegende Erfindung wird durch die nachfolgenden Beispiele illustriert.

### Beispiel 1 (Herstellung der wässrige Lösung des Calciumsalzes von (6S)-N(5)-Methyl-5,6,7,8-tetrahydrofolsäure der Formel III)

100 g (6S)-5,6,7,8-Tetrahydrofolsäure der Formel II mit einer Diastereoisomerenreinheit von (6S):(6R) von 92:8 - hergestellt gemäss EP 0 600 460 - wurden in Wasser, welches eine Temperatur von 7°C hatte, unter einer Stickstoffatmosphäre suspendiert.

Die Verbindung der Formel II wurde durch die Hinzugabe von 65 ml 20%-iger (w/w) wässriger NaOH Lösung aufgelöst. Der pH-Wert betrug dann 9,05.

Zu dieser Lösung wurden 23,7 g 36%-ige (w/w) wässrige Formaldehydlösung innerhalb von 5 Minuten bei einer Temperatur von 8°C unter Rühren hinzugegeben.

Nach 15 Minuten wurde eine wässrige NaBH₄-Lösung, hergestellt durch Auflösen von 21,3 g NaBH₄ in 50 ml Wasser und 1 ml 20%-iger (w/w) wässriger NaOH Lösung, während 1 Stunde bei einer Temperatur von 8°C unter Rühren hinzugegeben.

Dieses Gemisch wurde während 30 Minuten bei einer Temperatur von 8°C und anschliessend während 20 Minuten bei einer Temperatur von 61°C gerührt.

Die Reaktionstemperatur wurde dann innerhalb von 2 Stunden auf 20°C erniedrigt.

Dann wurden 79 ml 18%-ige (w/w) wässriger HCl Lösung tropfenweise hinzugegeben. Der pH-Wert betrug dann 8,03.

Das Gemisch wurde auf eine Temperatur 4°C abgekühlt, um die Borat-Ausfällung zu ermöglichen. Nach 2 Stunden wurden die Borate durch Filtration entfernt.

Der pH-Wert der resultierenden Lösung wurde durch die Hinzugabe von 10 ml 18%-ige (w/w) wässriger HCl Lösung auf einen Wert von 7,03 eingestellt.

Diese Lösung wurde auf eine Temperatur von 20°C erwärmt, und dann wurden zuerst 1,62 g an Di-natrium-EDTA und dann 0,82 Äquivalente (25,44 g) an CaCl₂.2H₂O hinzugegeben.

Dann wurde der pH-Wert auf einen Wert von 6,9 durch die Hinzugabe von 2 ml 20%-iger (w/w) wässriger NaOH Lösung eingestellt.

Die selektive Kristallisation des Calciumsalzes von (6RS)-N(5)-Methyl-5,6,7,8-tetrahydrofolsäure erfolgte durch Animpfen der Lösung mit 100 mg von vorgängig hergestellten Kristallen des Calciumsalzes von (6RS)-N(5)-Methyl-5,6,7,8-tetrahydrofolsäure, gefolgt von einer Temperaturerniedrigung von Raumtemperatur auf eine Temperatur von 4°C innerhalb von 40 Minuten.

Die resultierende Suspension wurde unter Rühren während 18 Stunden bei einer Temperatur von 4°C gehalten.

Die erhaltenen Kristalle wurden abfiltriert.

Die so erhaltene wässrige Lösung des Calciumsalzes von (6S)-N(5)-Methyl-5,6,7,8-tetrahydrofolsäure der Formel III hatte einem Anteil des entsprechenden (6R)-Diastereoisomers von ≤ 2 Gew.-%, bestimmt mittels HPLC an einer chiralen Säule.

### Beispiel 2 (Herstellung von kristalliner (6S)-N(5)-Methyl-5,6,7,8-tetrahydrofolsäure der Formel IV)

Zur wässrigen Lösung des Calciumsalzes von (6S)-N(5)-Methyl-5,6,7,8-tetrahydrofolsäure der Formel III mit einem Anteil des entsprechenden (6R)-Diastereoisomers von ≤ 2 Gew.-%, hergestellt gemäss obigem Beispiel 1, wurden innerhalb von 20 Minuten 6 ml 100% Essigsäure hinzugegeben, bis ein pH-Wert von 5,5 erhalten wurde.

Danach wurde die Temperatur auf 45°C erhöht.

Bei dieser Temperatur wurden nacheinander 1,35 g Natrium-dithionit und 1,9 g Di-natrium-EDTA hinzugegeben.

Zu dieser Lösung wurden 25 ml 100% Essigsäure innerhalb von 15 Minuten hinzugegeben.

Bei einem pH-Wert von 4,5 begann die Kristallisation von (6S)-N(5)-Methyl-5,6,7,8-tetrahydrofolsäure der Formel IV.

Der pH-Wert wurde im Bereich von 4,3 bis 4,4 durch kontinuierliche Hinzugabe von 100% Essigsäure (10 ml) gehalten.

Diese Suspension wurde 30 Minuten gerührt und anschliessend abfiltriert.

Der so erhaltene kristalline Festkörper wurde mit Wasser, welches eine Temperatur von 40°C hatte, gewaschen.

Es wurden 106,9 g an feuchtem kristallinen Festkörper erhalten.

Zur Bestimmung der Reinheit und des Verhältnisses der Diastereoisomeren wurde eine Probe der Verbindung der Formel IV mit 94%-igem (v/v) wässrigem Ethanol gewaschen und dann unter reduziertem Druck getrocknet. Dabei wurden folgende Resultate erhalten:
HPLC Reinheit: 96,45 %

Verhältnis von (6S)/(6R) = 99,1 : 0,9 (bestimmt mittels chiraler HPLC).

Das so erhaltene Produkt der Formel IV hatte die folgenden Stabilitätsdaten (gelagert bei einer Temperatur von 4°C):

| Zeit (Monate) | HPLC Reinheit (%) |
|---|---|
| 0 | 96.45 |
| 6 | 96.66 |

## Patentansprüche

1. Stabile, kristalline (6S)-N(5)-Methyl-5,6,7,8-tetrahydrofolsäure mit einer diastereoisomeren Reinheit von wenigstens 99%.

2. Stabile, kristalline (6S)-N(5)-Methyl-5,6,7,8-tetrahydrofolsäure nach Anspruch 1, **dadurch gekennzeichnet, dass** sie
- das in Figur 2a gezeigte "Differential Scanning Calorimetric" Profil, DSC,
- das in Figur 2b gezeigte thermogravimetrische Profil, TGA,
- das in Figur 3a gezeigte Pulverröntgendiagramm, und
- die in Figur 3b gezeigten 2 Theta-Werte mit einem Fehler von etwa ± 0,2 Grad
hat.

3. Stabile, kristalline (6S)-N(5)-Methyl-5,6,7,8-tetrahydrofolsäure nach einem der Ansprüche 1 bis 2, **dadurch gekennzeichnet, dass** das entsprechende Pulverröntgendiagramm die folgenden charakteristischen 2 Theta-Werte aufweist:
15,0
15,6
17,4
18,7
19,9
22,2
24,3 und
29,5.

4. Verfahren zur Herstellung von stabiler, kristalliner (6S)-N(5)-Methyl-5,6,7,8-tetrahydrofolsäure ,
**dadurch gekennzeichnet, dass** man
- zu einer wässrigen Lösung des Calciumsalzes von (6S)-N(5)-Methyl-5,6,7,8-tetrahydrofolsäure mit einem Anteil des entsprechenden (6R)-Diastereoisomers von ≤ 2 Gew.-% entweder Essigsäure oder eine Sulfonsäure in einer solchen Menge hinzugibt, bis ein pH-Wert von 5,5 erhalten wird,
- die so erhaltene Lösung auf eine Temperatur im Bereich von 44°C bis 46°C erwärmt,
- zu dieser erwärmten Lösung entweder Essigsäure oder eine Sulfonsäure in einer solchen Menge hinzugibt, bis ein pH-Wert im Bereich von 4,3 bis 4,4 erhalten wird, wobei die Kristallisation von (6S)-N(5)-Methyl-5,6,7,8-tetrahydrofolsäure beginnt, wobei während dieser Kristallisation der pH-Wert im Bereich von 4,3 bis 4,4 durch kontinuierliche Hinzugabe von entweder Essigsäure oder einer Sulfonsäure aufrecht erhalten wird, und
- den so erhaltenen kristallinen Festkörper bei einer Temperatur im Bereich von 44°C bis 46°C abfiltriert und isoliert.

5. Verfahren nach Anspruch 4, **dadurch gekennzeichnet, dass** man den erhaltenen kristallinen Festkörper mit Wasser, welches eine Temperatur von etwa 40°C hat, wäscht.

6. Verfahren nach einem der Ansprüche 4 bis 5, **dadurch gekennzeichnet, dass** die Sulfonsäure p-Toluolsulfonsäure ist.

7. Verfahren nach einem der Ansprüche 4 bis 6, **dadurch gekennzeichnet, dass** man die wässrige Lösung des Calciumsalzes von (6S)-N(5)-Methyl-5,6,7,8-tetrahydrofolsäure mit einem Anteil des entsprechenden (6R)-Diastereoisomers von ≤ 2 Gew.-% gemäss einem Verfahren herstellt, bei welchem
man (6S)-5,6,7,8-Tetrahydrofolsäure mit einem Anteil des entsprechenden (6R)-Diastereoisomers im Bereich von 4 Gew.-% bis 8 Gew.-% in Wasser methyliert,
wobei man
- zum erhaltenen methylierten Reaktionsgemisch von 0,70 bis 0,82 Äquivalente, bezogen auf die eingesetzte Menge an THF, an Calciumchlorid hinzugibt,
- aus der erhaltenen wässrigen Lösung das Calciumsalz von (6RS)-N(5)-Methyl-5,6,7,8-tetrahydrofolsäure selektiv auskristallisiert und abtrennt, und
- die wässrige Lösung des Calciumsalzes von (6S)-N(5)-Methyl-5,6,7,8-tetrahydrofolsäure mit einem Anteil des entsprechenden (6R)-Diastereoisomers von ≤ 2 Gew.-% gewinnt.

8. Verfahren nach Anspruch 7, **dadurch gekennzeichnet, dass** das Calciumchlorid in fester Form oder in der Form einer wässrigen Lösung hinzugegeben wird.

9. Verfahren nach einem der Ansprüche 7 bis 8, **dadurch gekennzeichnet, dass** die selektive Kristallisation des Calciumsalzes von (6RS)-N(5)-Methyl-5,6,7,8-tetrahydrofolsäure durch Animpfen mit vorgängig hergestellten Kristallen des Calciumsalzes von (6RS)-N(5)-Methyl-5,6,7,8-tetrahydrofolsäure erfolgt, gefolgt von einer Temperaturerniedrigung von Raumtemperatur auf eine Temperatur im Bereich von 3°C bis 5°C, und dass diese Temperatur während 16 bis 18 Stunden aufrecht gehalten wird.

10. Verwendung der stabilen, kristallinen (6S)-N(5)-Methyl-5,6,7,8-tetrahydrofolsäure nach einem der Ansprüche 1 bis 3 zur Herstellung eines Nährungsergänzungsmittels oder eines Medikaments
- für die Behandlung und/oder Bekämpfung von menschlichen und/oder tierischen Tumoren und/oder
- für die synergistische Beeinflussung einer Krebs bekämpfenden Verbindung und/oder
- für die Verminderung der Toxizität einer Krebs bekämpfenden Verbindung und/oder
- zum Schutz menschlicher und/oder tierischer Zellen.

## Claims

1. Stable, crystalline (6S)-N(5)-methyl-5,6,7,8-tetrahydrofolic acid having a diastereoisomeric purity of at least 99%.

2. Stable, crystalline (6S)-N(5)-methyl-5,6,7,8-tetrahydrofolic acid according to claim 1, **characterized in that** it has
- the "differential scanning calorimetric" profile, DSC, as shown in Figure 2a,
- the thermo gravimetric profile, TGA, as shown in Figure 2b,
- the X-ray powder diffraction diagram as shown in Figure 3a, and
- the 2 Theta-values having an error of about + 0.2 degree as shown in Figure 3b.

3. Stable, crystalline (6S)-N(5)-methyl-5,6,7,8-tetrahydrofolic acid according to one of claims 1 to 2, **characterized in that** the corresponding X-ray powder diffraction diagram has the following characteristic 2 Theta-values:
15.0
15.6
17.4
18.7
19.9
22.2
24.3 and
29.5.

4. A process for the preparation of stable, crystalline (6S)-N(5)-methyl-5,6,7,8-tetrahydrofolic acid,
**characterized in that**
- to an aqueous solution of the calcium salt of (6S)-N(5)-methyl-5,6,7,8-tetrahydrofolic acid having a content of the corresponding (6R)-diastereoisomer of ≤ 2% by weight is added either acetic acid or a sulfonic acid in such an amount, until a pH-value of 5.5 is obtained,
- the so obtained solution is warmed to a temperature in the range from 44°C to 46°C,
- to this warmed solution is added either acetic acid or a sulfonic acid in such an amount, until a pH-value in the range from 4.3 to 4.4 is obtained, whereby the crystallization of (6S)-N(5)-methyl-5,6,7,8-tetrahydrofolic acid begins, whereby during this crystallization the pH-value is maintained in the range from 4.3 to 4.4 by continuous addition of either acetic acid or of a sulfonic acid, and
- the so obtained crystalline solid is filtered off at a temperature in the range from 44°C to 46°C and is isolated.

5. The process according to claim 4, **characterized in that** the obtained crystalline solid is washed with water having a temperature of about 40°C.

6. The process according to one of claims 4 to 5, **characterized in that** the sulfonic acid is p-toluene sulfonic acid.

7. The process according to one of claims 4 to 6, **characterized in that** the aqueous solution of the calcium salt of (6S)-N(5)-methyl-5,6,7,8-tetrahydrofolic acid having a content of the corresponding (6R)-diastereoisomer of ≤ 2% by weight is prepared according to a process, whereby
(6S)-5,6,7,8-tetrahydrofolic acid having a content of the corresponding (6R)-diastereoisomer in the range from 4% by weight to 8% by weight is methylated in water, whereby
- to the obtained methylated reaction mixture are added from 0.70 to 0.82 equivalents, based on the amount of THF, of calcium chloride,
- from the obtained aqueous solution the calcium salt of (6RS)-N(5)-methyl-5,6,7,8-tetrahydrofolic acid is selectively crystallized and separated, and
- the aqueous solution of the calcium salt of (6S)-N(5)-methyl-5,6,7,8-tetrahydrofolic acid having a content of the corresponding (6R)-diastereoisomer of ≤ 2% by weight is obtained.

8. The process according to claim 7, **characterized in that** the calcium chloride is added in solid form or in the form of an aqueous solution.

9. The process according to one of claims 7 to 8, **characterized in that** the selective crystallization of the calcium salt of (6RS)-N(5)-methyl-5,6,7,8-tetrahydrofolic acid is realized by seeding with previously prepared crystals of the calcium salt of (6RS)-N(5)-methyl-5,6,7,8-tetrahydrofolic acid, followed by a lowering of the temperature from room temperature to a temperature in the range from 3°C to 5°C, and that this temperature is kept during 16 to 18 hours.

10. Use of the stable, crystalline (6S)-N(5)-methyl-5,6,7,8-tetrahydrofolic acid according to one of claims 1 to 3 for the preparation of a dietary supplement or of a medicament
- for the treatment and/or the control of human and/or animal tumors and/or
- for the synergistic exertion of influence of a cancer controlling compound and/or
- for the reduction of the toxicity of a cancer controlling compound and/or
- for the protection of human and/or animal cells.

## Revendications

1. Acide (6S)-N(5)-méthyl-5,6,7,8-tétrahydrofolique cristallin stable présentant une pureté diastéréoisomérique d'au moins 99 %.

2. Acide (6S)-N(5)-méthyl-5,6,7,8-tétrahydrofolique cristallin stable selon la revendication 1, **caractérisé en ce qu'**il possède
- le profil *"Differential Scanning Calorimetric"* (DSC) présenté à la figure 2a,
- le profil thermogravimétrique (TGA) présenté à la figure 2b,
- le diffractogramme de rayons X sur poudre présenté à la figure 3a,
- les valeurs deux-thêta présentées à la figure 3b avec une erreur d'environ ± 0,2 degré.

3. Acide (6S)-N(5)-méthyl-5,6,7,8-tétrahydrofolique cristallin stable selon l'une des revendications 1 à 2, **caractérisé en ce que** le diffractogramme de rayons X sur poudre correspondant présente les valeurs deux-thêta caractéristiques suivantes:
15,0
15,6
17,4
18,7
19,9
22,2
24,3 et
29,5

4. Procédé de préparation d' acide (6S)-N(5)-méthyl-5,6,7,8-tétrahydrofolique cristallin stable,
**caractérisé en ce que** l'on
- ajoute à une solution aqueuse du sel de calcium de l'acide (6S)-N(5)-méthyl-5,6,7,8-tétrahydrofolique présentant une proportion du diastéréoisomère (6R) correspondant inférieure ou égale à 2 % en poids, soit de l'acide acétique, soit un acide sulfonique, dans une proportion telle qu'un pH de 5,5 soit obtenu,
- chauffe la solution ainsi obtenue à une température située dans l'intervalle de 44 à 46 °C,
- ajoute à cette solution chauffée, soit de l'acide acétique, soit un acide sulfonique dans une proportion telle qu'elle permette d'obtenir un pH situé dans l'intervalle de 4,3 à 4,4, auquel la cristallisation de l'acide (6S)-N(5)-méthyl-5,6,7,8-tétrahydrofolique commence, ce pH situé dans l'intervalle de 4,3 à 4,4 étant maintenu durant cette cristallisation par l'adjonction continue, soit d'acide acétique, soit d'un acide sulfonique et
- sépare par filtration et isole le solide cristallin ainsi obtenu à une température comprise dans l'intervalle de 44 à 46 °C.

5. Procédé selon la revendication 4, **caractérisé en ce que** l'on lave le solide cristallin obtenu avec de l'eau, qui présente une température d'environ 40 °C.

6. Procédé selon l'une des revendications 4 à 5, **caractérisé en ce que** l'acide sulfonique est l'acide p-toluène-sulfonique.

7. Procédé selon l'une des revendications 4 à 6, **caractérisé en ce que** l'on prépare la solution aqueuse du sel de calcium de l'acide (6S)-N(5)-méthyl-5,6,7,8-tétrahydrofolique présentant une proportion du diastéréoisomère (6R) correspondant inférieure ou égale à 2 % en poids, selon un procédé dans lequel
on méthyle dans de l'eau l'acide (6S)-5,6,7,8-tétrahydrofolique présentant une proportion du diastéréoisomère (6R) correspondant comprise dans l'intervalle de 4 à 8 % en poids,
- 0,70 à 0,82 équivalent de chlorure de calcium par rapport à la proportion de THF mise en oeuvre étant ajouté au mélange réactionnel méthylé obtenu,
- le sel de calcium de l'acide (6RS)-N(5)-méthyl-5,6,7,8-tétrahydrofolique étant sélectivement cristallisé à partir de la solution aqueuse obtenue et séparé, et
- la solution aqueuse du sel de calcium de l'acide (6S)-N(5)-méthyl-5,6,7,8-tétrahydrofolique présentant une proportion du diastéréoisomère (6R) correspondant inférieure ou égale à 2 % en poids étant extraite.

8. Procédé selon la revendication 7, **caractérisé en ce que** le chlorure de calcium est ajouté sous forme solide ou en solution aqueuse.

9. Procédé selon l'une des revendications 7 à 8, **caractérisé en ce que** la cristallisation sélective du sel de calcium de l'acide (6RS)-N(5)-méthyl-5,6,7,8-tétrahydrofolique est opérée par ensemencement avec des cristaux préalablement préparés du sel de calcium de l'acide (6RS)-N(5)-méthyl-5,6,7,8-tétrahydrofolique, suivi d'un abaissement de la température, passant de la température ambiante à une température située dans l'intervalle de 3 à 5 °C, et que cette température est maintenue pendant 16 à 18 heures.

10. Utilisation de l'acide (6S)-N(5)-méthyl-5,6,7,8-tétrahydrofolique cristallin stable selon l'une des revendications 1 à 3 pour la préparation d'un complément alimentaire ou d'un médicament
- pour le traitement des tumeurs humaines et/ou animales et/ou la lutte contre celles-ci et/ou
- pour la potentialisation synergique de l'action d'un composé anticancéreux et/ou
- pour la diminution de la toxicité d'un composé anticancéreux et/ou
- pour la protection des cellules humaines et/ou animales.
